# EUROPÄISCHE PATENTSCHRIFT

(11) **EP 3 618 891 B1**
(45) Veröffentlichungstag und Bekanntmachung des Hinweises auf die Patenterteilung: **06.11.2024**
(21) Anmeldenummer: 18719512.8
(22) Anmeldetag: 18.04.2018
(51) Int. Cl.: A61M 5/168, A61M 5/14

(54) **TROPFKAMMERANORDNUNG FÜR EIN MEDIZINISCHES INFUSIONSSYSTEM**
DRIP CHAMBER ARRANGEMENT FOR A MEDICAL INFUSION SYSTEM
CHAMBRE GOUTE-A-GOUTE POUR SYSTÈME D'INJECTION MÉDICAL

(30) Priorität: 04.05.2017 DE 202017002518 U
(43) Veröffentlichungstag der Anmeldung: 11.03.2020
(73) Patentinhaber: B. Braun Melsungen AG, 34212 Melsungen (DE)
(72) Erfinder: FUCHS, Jürgen, 34308 Bad Emstal (DE)
(74) Vertreter: Patentanwälte Ruff, Wilhelm, Beier, Dauster & Partner mbB
(86) Internationale Anmeldenummer: PCT/EP2018/059923
(87) Internationale Veröffentlichungsnummer: WO 2018/202424

(56) Entgegenhaltungen:
- EP-A1- 1 312 388
- EP-A1- 1 731 185
- EP-A1- 1 747 787
- WO-A1-2012/038847
- WO-A1-91/01155
- US-A- 5 098 408
- US-A1- 2011 125 103

## Beschreibung

Die Erfindung betrifft eine Tropfkammeranordnung nach dem Oberbegriff des Anspruchs 1.

Eine Tropfkammeranordnung ist im Bereich der Medizintechnik allgemein bekannt und zur Verwendung bei der Infusionstherapie vorgesehen. Die bekannte Tropfkammeranordnung weist ein Tropfkammergehäuse auf. An einen Stirnende des Tropfkammergehäuses ist ein Einstechdorn mit einer Durchgangsbohrung angeordnet. Der Einstechdorn ist zum Einstechen in einen eine Infusionsflüssigkeit beinhaltenden Infusionsbehälter vorgesehen. Über die Durchgangsbohrung des Einstechdorns kann Infusionsflüssigkeit aus dem Infusionsbehälter in das Tropfkammergehäuse geleitet werden. Die Infusionsflüssigkeit ist über einen bodenseitig angeordneten Durchlass aus dem Tropfkammergehäuse ableitbar. In Verbindung mit einem fluidleitend mit dem Durchlass verbundenen Infusionsschlauch und ggfs. weiteren Bauteilen bildet die Tropfkammeranordnung ein bekanntes medizinisches Infusionssystem. Um einen Volumenstrom der Infusionsflüssigkeit - der hier im speziellen auch als Tropfrate bezeichnet wird - durch das Infusionssystem an medizinische Erfordernisse anpassen zu können, weisen bekannte Infusionssysteme eine Rollenklemme auf. Die Rollenklemme ist als zu der Tropfkammeranordnung separates Bauteil vorgesehen und steht in einer Klemmverbindung mit einem Schlauchmantelabschnitt des Infusionsschlauches. Der wirksame Querschnitt des Infusionsschlauches kann in Abhängigkeit des Klemmdrucks der Rollenklemme verändert werden. Auf diese Weise wird eine grobe Einstellung des Volumenstroms erreicht.

Aus der US 5 098 408 A ist eine Tropfkammeranordnung mit einem Tropfkammergehäuse und einer Stelleinrichtung zur Einstellung eines aus dem Tropfkammergehäuse abzuleitenden Volumenstroms einer Infusionsflüssigkeit bekannt. Die Stelleinrichtung ist relativ zu dem Tropfkammergehäuse drehbar an diesem befestigt. Mittels einer Drehung der Stelleinrichtung ist ein zur Ableitung der Infusionsflüssigkeit vorgesehener Fluidleitungskanal zur Einstellung des Volumenstroms veränderlich.

Aus der EP 1 747 787 A1 ist eine Tropfkammer mit einem integrierten Flussregler bekannt. Der Flussregler ist an einem Einlass der Tropfkammer angeordnet und weist einen ersten Körper mit einem Einlass und einen zweiten Körper mit einem Auslass auf. Die beiden Körper sind zur Einstellung einer Flussrate relativ zueinander drehbeweglich.

Die EP 1 731 185 A1 liefert eine detaillierte Beschreibung des Aufbaus des in der EP 1 747 787 A1 gezeigten Flussreglers.

Ähnliche Flussregler zur Einstellung eines Volumenstroms innerhalb eines medizinischen Infusionssystems sind aus der WO 2012/038847 A1, der WO 91/01155 A1 und der EP 1 312 388 A1 bekannt. Die dort gezeigten Volumenstromregler sind für eine manuelle Drehbetätigung zur Einstellung des Volumenstroms eingerichtet. Mittels der Drehbetätigung wird ein zur Ableitung des Volumenstroms vorgesehener Fluidleitungskanal in seiner wirksamen Länge und/oder in seinem wirksamen Querschnitt verändert.

Aufgabe der Erfindung ist es, eine Tropfkammeranordnung sowie ein medizinisches Infusionssystem der eingangs genannten Art zu schaffen, die eine verbesserte Einstellbarkeit des Volumenstromes erreichen.

Diese Aufgabe wird durch eine Tropfkammeranordnung mit den Merkmalen des Anspruchs 1 gelöst. Durch die erfindungsgemäße Lösung kann der Volumenstrom unmittelbar an der Tropfkammeranordnung eingestellt werden. Die Einstellbarkeit der Tropfrate erfolgt und ist somit unabhängig von den Eigenschaften eines Infusionsschlauches. Zudem ist die an dem Tropfkammergehäuse angeordnete Stelleinrichtung während der Dauer der Infusionstherapie außerhalb der Reichweite eines Patienten angeordnet. Demzufolge wird einem unbeabsichtigten oder beabsichtigten Verstellen der Stelleinrichtung und damit der Tropfrate vorgebeugt. Auf diese Weise wird eine verbesserte Einstellbarkeit des Volumenstromes erreicht. Die Stelleinrichtung ist unmittelbar an dem Tropfkammergehäuse befestigt und ist durch einen Kontakt zwischen einem Wandabschnitt des Tropfkammergehäuses und einem Wandabschnitt der Stelleinrichtung in axialer und/oder in radialer Richtung festgelegt.

Die in dieser Beschreibung verwendeten Begriffe unten, oben, axial, radial oder dergleichen beziehen sich auf eine gedachte Orientierung der Tropfkammeranordnung im Raum, bei der die Längsachse des Tropfkammergehäuses parallel zur Raumhochachse ausgerichtet ist und bei der die bei einer Infusionstherapie übliche Durchflussrichtung durch das Tropfkammergehäuse in Bezug auf die Raumhochachse von oben nach unten verläuft.

Gemäß der Erfindung ist die Stelleinrichtung relativ zu dem Tropfkammergehäuse drehbar an diesem befestigt. Durch diese Ausgestaltung der Erfindung wird eine besonders einfache Handhabbarkeit und Relativpositionierung der Stelleinrichtung erreicht.

Weiter gemäß der Erfindung ist die Stelleinrichtung derart gestaltet, dass ein dem Durchlass zugeordneter und fluidleitend mit diesem verbundener Fluidleitungskanal innerhalb der Tropfkammeranordnung in Abhängigkeit von der Relativposition der Stelleinrichtung veränderbar ist. Vorteilhafterweise ist derart die Geometrie des Fluidleitungskanals, insbesondere hinsichtlich seiner der Länge, seiner Breite und/oder seiner Tiefe, veränderbar. Wird beispielsweise der wirksame Querschnitt des Fluidleitungskanals mittels der Stelleinrichtung verengt oder geweitet, bewirkt dies eine Reduzierung bzw. eine Erhöhung der Tropfrate. Der derart veränderbare Fluidleitungskanal ist vorteilhafterweise zwischen dem Durchlass und einem Austritt, der zum Ausströmen der Infusionsflüssigkeit aus der Tropfkammeranordnung vorgesehen ist, angeordnet. Der Fluidleitungskanal kann zumindest abschnittsweise in einem Wandabschnitt der Stelleinrichtung und/oder in einem Wandabschnitt des Tropfkammergehäuses ausgebildet sein. Alternativ oder zusätzlich kann der Fluidleitungskanal zumindest einen Abschnitt eines zwischen der Stelleinrichtung und dem Tropfkammergehäuse ausgebildeten Spalts umfassen.

In weiterer Ausgestaltung der Erfindung umfasst der Fluidleitungskanal einen Abschnitt einer Drosselnut, die einen längs des Nutverlaufs veränderlichen Nutquerschnitt aufweist. Die Drosselnut ist in einem Wandabschnitt der Stelleinrichtung eingebracht. Die Drosselnut ist derart angeordnet, dass eine Veränderung der Relativposition der Stelleinrichtung eine Veränderung der Positionierung des Durchlasses längs der Drosselnut bewirkt. Da die Drosselnut einen veränderlichen Nutquerschnitt aufweist ist somit der wirksame Querschnitt des Fluidleitungskanals veränderbar. Alternativ oder zusätzlich ist derart zudem die wirksame Länge, d.h. die tatsächlich durchströmbare Länge, des Fluidleitungskanals in Abhängigkeit von der Relativposition der Stelleinrichtung veränderbar.

In weiterer Ausgestaltung der Erfindung weist die Drosselnut eine längs des Nutverlaufs veränderliche Nuttiefe auf. Diese Ausgestaltung der Erfindung erlaubt eine besonders toleranzgerechte Herstellung des veränderlichen Nutverlaufs. Demzufolge kann eine besonders präzise Einstellbarkeit der Tropfrate erreicht werden.

In weiterer Ausgestaltung der Erfindung ist die Drosselnut im Wesentlichen senkrecht zur Längsachse des Tropfkammergehäuses orientiert und weist einen im Wesentlichen kreisbogenförmigen Verlauf auf. Durch die derartige Ausrichtung der Drosselnut ergibt sich demnach - ausgehend von einer funktionsüblichen Ausrichtung der Tropfkammeranordnung - eine abschnittsweise horizontale Durchströmung der Tropfkammeranordnung. Vorteilhafterweise liegt das Zentrum des Kreisbogens auf der Rotationsachse der Stelleinrichtung, soweit diese relativ zu dem Tropfkammergehäuse drehbar ausgeführt ist. Weiter gemäß der Erfindung weist die Stelleinrichtung eine kreisbogenförmige Führungsnut auf, in der ein relativ zu dem Tropfkammergehäuse festgelegtes Führungselement geführt ist. Das Führungselement kann ein Stift, ein Zapfen, ein Nocken oder dergleichen sein. Durch die Führungsnut und das Führungselement wird eine besonders präzise Ausrichtung des Durchlasses gegenüber der Drosselnut erreicht.

In weiterer Ausgestaltung der Erfindung ist die Stelleinrichtung über eine Rastverbindung an einem Bodenabschnitt des Tropfkammergehäuses befestigt. Die Rastverbindung ist vorteilhafterweise derart gestaltet, dass die Stelleinrichtung gegenüber dem Tropfkammergehäuse drehbar ist. Zu diesen Zweck weist der Bodenabschnitt vorteilhafterweise einen in Umfangsrichtung des Tropfkammergehäuses erstreckten Ring oder einen Wulst auf, an dem zumindest eine an der Stelleinrichtung angeordnete Rastanordnung drehbeweglich verrastet ist.

In weiterer Ausgestaltung der Erfindung ist ein Dichtelement zwischen dem Tropfkammergehäuse und der Stelleinrichtung angeordnet. Das Dichtelement ist zur fluiddichten Abdichtung eines zwischen den Tropfkammergehäuse und der Stelleinrichtung ausgebildeten Spaltes vorgesehen.

In weiterer Ausgestaltung der Erfindung ist das Dichtelement derart gestaltet, dass es in axialer Richtung eine Federkraft auf die Stelleinrichtung und das Tropfkammergehäuse bewirkt. Vorteilhafterweise weist das Dichtelement eine gummielastische Materialzusammensetzung auf. Die Materialzusammensetzung des Dichtelements beinhaltet vorzugsweise Silikon. Durch die derartige axiale Vorspannung zwischen Tropfkammergehäuse und Stelleinrichtung wird eine spielfreie und somit toleranzgerechte Ausrichtung der beiden Bauteile gegeneinander erreicht.

Die der Erfindung zugrundeliegende Aufgabe wir für ein medizinisches Infusionssystem der eingangs genannten Art dadurch gelöst, dass die Tropfkammeranordnung nach den vorstehenden Ausführungen gestaltet ist, wobei das Tropfkammergehäuse über die Stelleinrichtung fluidleitend mit dem Infusionsschlauch verbunden ist. Demnach ist der Infusionsschlauch an einem seiner Stirnenden fluidleitend mit der Stelleinrichtung verbunden. An dem gegenüberliegenden Stirnende weist der Infusionsschlauch vorzugsweise einen Anschlusskonnektor zur Verbindung mit einem patientenseitigen Zugang auf.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt in schematischer Perspektivdarstellung eine Ausführungsform eines erfindungsgemäßen medizinischen Infusionssystems aufweisend eine Ausführungsform einer erfindungsgemäßen Tropfkammeranordnung,
- Fig. 2a bis c: jeweils in schematischer Schnittdarstellung einen Bodenbereich III des Infusionssystems nach Fig. 1 in einer bezüglich des Volumenstromes vollständig geöffneten Position (Fig. 2a), einer vollständig geschlossenen Position (Fig. 2b) sowie einer teilweise geöffneten Einstellposition der Stelleinrichtung (Fig. 2c),
- Fig. 3: in schematischer Darstellung eine Stelleinrichtung der Tropfkammeranordnung nach den Fig. 1 bis 2c entlang eines Schnittes D-D gemäß Fig. 2a und
- Fig. 4a bis c: in schematischer Darstellung jeweils einen Bereich einer Drosselnut der Stelleinrichtung nach Fig. 3 entlang eines Schnittes A-A (Fig. 4a), B-B (Fig. 4b) sowie C-C (Fig. 4c).

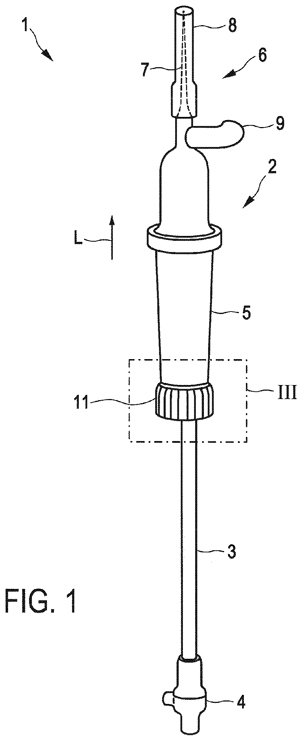

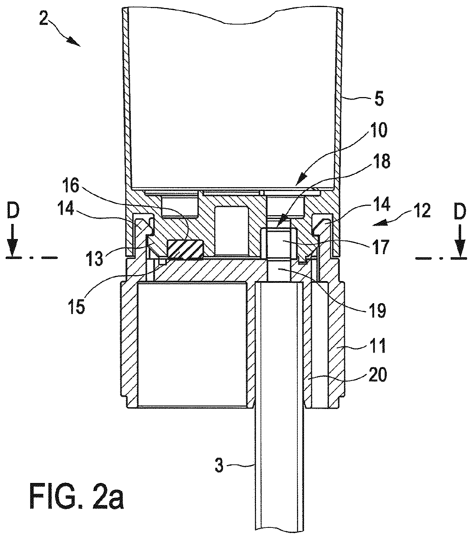

Ein medizinisches Infusionssystem 1 nach Fig. 1 ist für die Verwendung bei einer Infusionstherapie vorgesehen. Das medizinische Infusionssystem weist eine Tropfkammeranordnung 2 auf, deren Details anhand der Fig. 2a bis 4c ersichtlich sind. Das medizinische Infusionssystem 1 weist zudem einen Infusionsschlauch 3 auf, der an seinem der Tropfkammeranordnung 2 abgewandten Stirnende einen Anschlusskonnektor 4 zur Verbindung mit einem nicht näher ersichtlichen patientenseitigen Zugang aufweist.

Die Tropfkammeranordnung 2 weist ein im Wesentlichen zylindrisch ausgestaltetes Tropfkammergehäuse 5 auf, das in Bezug auf die Längsrichtung L in einem oberen Bereich ein Einstechteil 6 mit einem Einstechdorn 7 aufweist. Zum Schutz vor Beschädigungen und der Vermeidung von Verletzungen bei der Handhabung ist der Einstechdorn 7 von einer Abdeckkappe 8 abgedeckt. Der Einstechdorn 7 weist eine nicht näher ersichtliche Durchgangsbohrung auf und ist zum Einstechen in einen eine Infusionsflüssigkeit beinhaltenden Infusionsbehälter vorgesehen. Über die Durchgangsbohrung des Einstechdorns 7 kann Infusionsflüssigkeit aus dem nicht näher ersichtlichen Infusionsbehälter in das Tropfkammergehäuse 5 geleitet werden. Zur Belüftung des Infusionsbehälters bei der Ausleitung von Infusionsflüssigkeit weist die Tropfkammeranordnung 2 eine Belüftungseinheit 9 auf. In einem Bodenbereich III des Tropfkammergehäuses 5 ist ein Durchlass 10 zur Ableitung von Infusionsflüssigkeit aus dem Tropfkammergehäuse 5 vorgesehen. Der Durchlass 10 ist als im Wesentlichen parallel zur Längsrichtung L, d.h. axial, erstreckte Durchgangsöffnung in einem unteren Wandabschnitt des Tropfkammergehäuses 5 ausgebildet.

Weiter weist die Tropfkammeranordnung 2 eine Stelleinrichtung 11 auf, die zur Einstellung des Volumenstroms durch den Durchlass 10 vorgesehen ist. Die Stelleinrichtung 11 ist über eine Rastverbindung 12 an dem Tropfkammergehäuse 5 befestigt und somit in axialer und radialer Richtung an dem Tropfkammergehäuse 5 festgelegt. Die Rastverbindung 12 wird einerseits durch einen stirnendseitig an dem Tropfkammergehäuse 5 angeordneten Wulst 13, der sich zumindest abschnittsweise in Umfangsrichtung des Tropfkammergehäuses 5 erstreckt, und andererseits durch Rastelemente 14 gebildet. Die Rastelemente 14 sind an einem in Bezug auf die Längsachse L oberen Stirnendbereich der Stelleinrichtung 11 angeordnet. Die Stelleinrichtung 11 ist insoweit relativ zu dem Tropfkammergehäuse 5 drehbar an diesem befestigt. Zwischen dem Tropfkammergehäuse 5 und der Stelleinrichtung 11 ist ein Dichtelement 15 angeordnet. In einem in Bezug auf die Zeichenebene der Fig. 2a linken Bereich ist das Dichtelement 15 in eine Aufnahmenut 16 des Tropfkammergehäuses 5 eingepasst. In einem rechten Bereich ist das Dichtelement 15 in dem Durchlass 10 in radialer und axialer Richtung festgelegt und weist einen koaxial zu dem Durchlass 10 erstreckten Durchlasskanal 17 auf. Durch das derart angeordnete Dichtelement 15 wird zum einen eine fluiddichte Abdichtung zwischen der Stelleinrichtung 11 und dem Tropfkammergehäuse 5 erreicht. Zudem bewirkt das Dichtelement 15 in axialer Richtung eine Federkraft auf die Stelleinrichtung 11 und das Tropfkammergehäuse 5. Auf diese Weise wird eine toleranzgerechte Ausrichtung dieser beiden Bauteile gegeneinander erreicht.

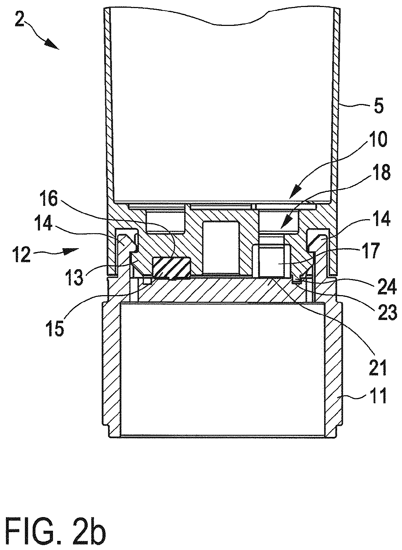

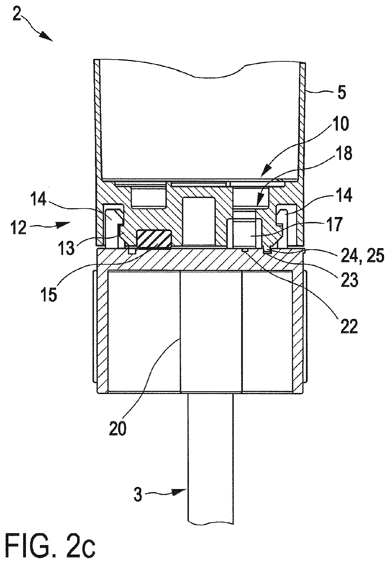

Wie anhand der Fig. 2a bis 2c ersichtlich ist, ist die Stelleinrichtung 11 relativ zu dem Tropfkammergehäuse 5 zwischen einer vollständig geöffneten Stellung (Fig. 2a) und einer vollständig geschlossenen Stellung (Fig. 2b) positionierbar, genauer: drehbar. Zwischen diesen beiden vorbeschriebenen Stellungen nimmt die Stelleinrichtung 11 eine beliebige Anzahl von Zwischenpositionen ein, wobei eine solche Zwischenposition exemplarisch in Fig. 2c ersichtlich ist. Auf diese Weise kann der Volumenstrom einer Infusionsflüssigkeit durch den Durchlass 10 in Abhängigkeit von der Relativposition der Stelleinrichtung 11 an medizinische Erfordernisse angepasst und insoweit eingestellt werden. Zu diesem Zweck wird je nach Relativposition der Stelleinrichtung 11 ein dem Durchlass 10 zugeordneter und fluidleitend mit diesem verbundener Fluidleitungskanal 18 innerhalb der Tropfkammeranordnung 2 verändert.

In der anhand Fig. 2a ersichtlichen vollständig geöffneten Position der Stelleinrichtung 11 ist der Durchlass 10 des Tropfkammergehäuses 5 fluidleitend mit Durchlasskanal 17 des Dichtelements 15 sowie mit einer in einem horizontal erstreckten Wandabschnitt 21 der Stelleinrichtung 11 angeordneten Durchgangsöffnung 19 verbunden. Der Fluidleitungskanal 18 umfasst in dieser Position der Stelleinrichtung 11 den Durchlasskanal 17 sowie die Durchgangsöffnung 19. Die Durchgangsöffnung 19 der Stelleinrichtung 11 ist fluidleitend mit dem Infusionsschlauch 3 verbunden, dessen Stirnendbereich zu diesem Zweck in einer Aufnahmehülse 20 der Stelleinrichtung 11 festgelegt ist.

Insbesondere ausgehend von der anhand Fig. 2a ersichtlichen Position der Stelleinrichtung 11 lässt sich diese in den anhand Fig. 2b ersichtlichen vollständig verschlossenen Zustand überführen. In diesem ist der Durchlasskanal 17 in axialer Richtung durch den Wandabschnitt 21 der Stelleinrichtung 11 verschlossen. In dieser Stellung der Stelleinrichtung 11 erfolgt somit kein Volumenstrom der Infusionsflüssigkeit durch den Durchlass 10.

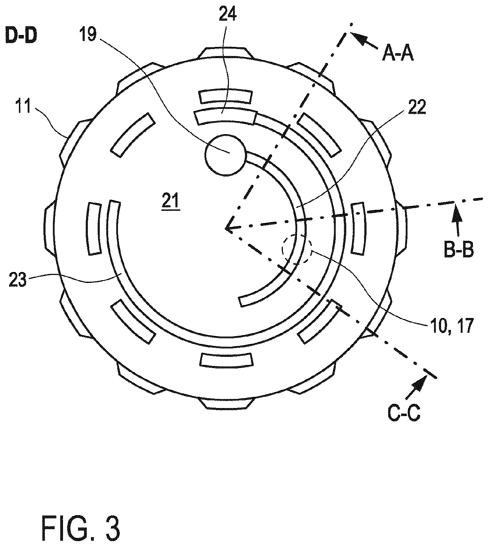

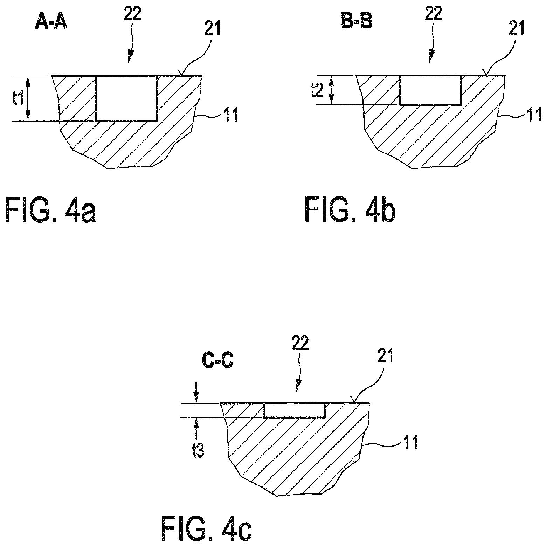

Um den Volumenstrom durch den Durchlass 10 stufenlos zwischen den beiden anhand der Fig. 2a und 2b ersichtlichen Stellungen einstellen zu können, weist die Stelleinrichtung 11 eine Drosselnut 22 auf. Die Drosselnut 22 ist in den oberen stirnendseitigen Wandabschnitt 21 der Stelleinrichtung 11 eingebracht und ist im Wesentlichen senkrecht zur Längsachse L des Tropfkammergehäuses 5 erstreckt. Weiter weist die Drosselnut 22 einen im Wesentlichen kreisbogenförmigen Verlauf auf. Der kreisbogenförmige Verlauf erstreckt sich über in etwa 180° und bildet somit in etwa einen Halbkreis. Wie insbesondere anhand der Fig. 4a bis 4c ersichtlich ist, weist die Drosselnut 22 einen längs des Nutverlaufs veränderlichen Nutquerschnitt auf. Im Bereich der Durchgangsöffnung 19 ist der Nutquerschnitt der Drosselnut 22 am größten, an dem der Durchgangsöffnung 19 abgewandten Ende der Drosselnut 22 am geringsten. Die Veränderung des Nutquerschnitts der Drosselnut 22 über deren Nutverlauf ist durch eine Änderung der Nuttiefe der Drosselnut 22 bewirkt. D.h. die Drosselnut 22 weist eine längs des Nutverlaufs veränderliche Nuttiefe auf. Wie anhand der Fig. 4a bis 4c ersichtlich ist, weist die Drosselnut 22 im Bereich der Durchgangsöffnung 19 eine Nuttiefe t1, in einem mittleren Längenbereich eine Nuttiefe t2 und in einem hinteren Längenbereich eine Nuttiefe t3 auf. In der anhand Fig. 2c ersichtlichen Relativposition der Stelleinrichtung 11 ist ein Abschnitt der Drosselnut 22 in Bezug auf die Längsachse L unterhalb des Durchlasses 10 des Tropfkammergehäuses 5 und somit auch unterhalb des Durchlasskanals 17 des Dichtelements 15 angeordnet. Zur besseren Verdeutlichung der Zusammenhänge sind der Durchlass 10 und der Durchlasskanal 17 in Fig. 3 strichliert dargestellt. In dieser Relativposition der Stelleinrichtung 11 umfasst der Fluidleitungskanal 18 somit auch einen Abschnitt der Drosselnut 22, nämlich den Abschnitt, der sich fluidleitend zwischen der Durchgangsöffnung 19 und dem Durchlass 10 erstreckt (Fig. 3). Somit ist die Geometrie des Fluidleitungskanals 18 in Abhängigkeit von der Relativposition der Stelleinrichtung 11, genauer: in Abhängigkeit von ihrer Drehposition gegenüber dem Tropfkammergehäuse 5, veränderbar. In der anhand Fig. 2c und Fig. 3 ersichtlichen Stellung der Stelleinrichtung 11 durchströmt die Infusionsflüssigkeit zunächst den Durchlass 10, sodann den Durchlasskanal des Dichtelements 15 und wird weiter ausgehend von diesem in die Drosselnut 22 eingeleitet. Die Infusionsflüssigkeit 22 ergießt sich längs der Drosselnut 22 in Richtung der Durchgangsöffnung 19 der Stelleinrichtung 11 und verlässt durch diese die Tropfkammeranordnung 2. Je nach Stellung der Stelleinrichtung 11 ist der Fluidleitungskanal 18 somit zum einen hinsichtlich seiner Länge und zum anderen hinsichtlich seines wirksamen Querschnitts veränderbar. Letzteres, da die Drosselnut 22 einen längs ihres Nutverlaufs veränderlichen Nutquerschnitt aufweist (Fig. 4a bis 4c).

Um eine möglichst toleranzgerechte Ausrichtung der Stelleinrichtung 11 gegenüber dem Tropfkammergehäuse 5 zu ermöglichen, weist die Stelleinrichtung zudem eine Führungsnut 23 auf. Die Führungsnut 23 ist in den Wandabschnitt 21 der Stelleinrichtung eingebracht und weist einen kreisbogenförmigen Verlauf auf. Der kreisbogenförmige Verlauf der Führungsnut 23 ist konzentrisch zu dem kreisbogenförmigen Verlauf der Drosselnut 22 und erstreckt sich über in etwa 270°. In der Führungsnut 23 ist ein relativ zu dem Tropfkammergehäuse 5 festgelegtes Führungselement 24 geführt. Das Führungselement 24 ist in Form einer Haltenase 25 gestaltet. Die Haltenase 25 ist stirnendseitig an dem Wulst 13 des Tropfkammergehäuses 5 angeordnet.

## Patentansprüche

1. Tropfkammeranordnung (2) für ein medizinisches Infusionssystem (1) aufweisend ein Tropfkammergehäuse (5) mit einer Längsachse und einem Durchlass (10) zur Ableitung von Infusionsflüssigkeit aus dem Tropfkammergehäuse (5), und aufweisend eine Stelleinrichtung (11) zur Einstellung eines Volumenstromes der Infusionsflüssigkeit durch den Durchlass (10), die relativ zu dem Tropfkammergehäuse (5) positionierbar und in axialer und/oder radialer Richtung festgelegt an diesem befestigt ist, wobei die Stelleinrichtung (11) relativ zu dem Tropfkammergehäuse (5) um die Längsachse drehbar an diesem befestigt ist, und wobei die Stelleinrichtung derart gestaltet ist, dass ein dem Durchlass (10) zugeordneter und fluidleitend mit diesem verbundener Fluidleitungskanal (18) innerhalb der Tropfkammeranordnung (2) in Abhängigkeit von der relativen Drehposition der Stelleinrichtung (11) veränderbar ist, wobei der Fluidleitungskanal (18) einen Abschnitt einer Drosselnut (22) umfasst, die einen längs des Nutverlaufs veränderlichen Nutquerschnitt aufweist, **dadurch gekennzeichnet, dass** die Stelleinrichtung (11) eine kreisbogenförmige Führungsnut (23) aufweist, in der ein relativ zu dem Tropfkammergehäuse (5) festgelegtes Führungselement (24) geführt ist, und dass die kreisbogenförmige Führungsnut (23) und die Drosselnut (22) in einen stirnendseitigen oberen Wandabschnitt (21) der Stelleinrichtung (11) eingebracht sind.

2. Tropfkammeranordnung (2) nach Anspruch 1, **dadurch gekennzeichnet, dass** die Drosselnut (22) eine längs des Nutverlaufs veränderliche Nuttiefe (t1, t2, t3) aufweist.

3. Tropfkammeranordnung (2) nach Anspruch 1 oder 2, **dadurch gekennzeichnet, dass** die Drosselnut (22) im Wesentlichen senkrecht zur Längsachse (L) des Tropfkammergehäuses (5) orientiert ist und einen im Wesentlichen kreisbogenförmigen Verlauf aufweist.

4. Tropfkammeranordnung (2) nach einem der vorhergehenden Ansprüche, **dadurch gekennzeichnet, dass** die Stelleinrichtung (11) über eine Rastverbindung (12) an einem Bodenabschnitt des Tropfkammergehäuses (5) befestigt ist.

5. Tropfkammeranordnung (2) nach Anspruch 4, **dadurch gekennzeichnet, dass** die Rastverbindung (12) ausgebildet ist zwischen einem Wulst (13), der stirnendseitig unten an dem Tropfkammergehäuse (5) angeordnet ist und sich zumindest abschnittsweise in Umfangsrichtung des Tropfkammergehäuses (5) erstreckt, und mehreren Rastelementen (14), die an dem stirnendseitig oberen Wandabschnitt (21) der Stelleinrichtung (11) angeordnet und in Umfangsrichtung drehbeweglich mit dem Wulst (13) verrastet sind.

6. Tropfkammeranordnung (2) nach einem der vorhergehenden Ansprüche, **dadurch gekennzeichnet, dass** ein Dichtelement (15) zwischen dem Tropfkammergehäuse (5) und der Stelleinrichtung (11) angeordnet ist.

7. Tropfkammeranordnung (2) nach Anspruch 6, **dadurch gekennzeichnet, dass** das Dichtelement (15) derart gestaltet ist, dass es in axialer Richtung eine Federkraft auf die Stelleinrichtung (11) und das Tropfkammergehäuse (5) bewirkt.

8. Medizinisches Infusionssystem (1) mit einer Tropfkammeranordnung (2) und einem Infusionsschlauch (3), **dadurch gekennzeichnet, dass** die Tropfkammeranordnung (2) nach einem der vorhergehenden Ansprüche ausgeführt ist, wobei das Tropfkammergehäuse (5) über die Stelleinrichtung (11) fluidleitend mit dem Infusionsschlauch (3) verbunden ist.

## Claims

1. Drip chamber arrangement (2) for a medical infusion system (1) having a drip chamber housing (5) with a longitudinal axis and a passage (10) for discharging infusion fluid from the drip chamber housing (5), and having an adjusting device (11) for adjusting a volume flow of the infusion fluid through the passage (10), which can be positioned relative to the drip chamber housing (5) and is fixedly attached thereto in the axial and/or radial direction, wherein the adjusting device (11) is attached to the drip chamber housing (5) and rotatable around the longitudinal axis relative thereto, and wherein the adjusting device is designed in such a way that a fluid conduction channel (18) within the drip chamber arrangement (2), which is associated with the passage (10) and connected thereto in a fluid-conducting manner, can be varied as a function of the relative rotational position of the adjusting device (11), the fluid conduction channel (18) comprising a section of a throttle groove (22) which has a groove cross-section which varies along the course of the groove, **characterized in that** the adjusting device (11) has an arcuate guide groove (23) in which a guide element (24) fixed relative to the drip chamber housing (5) is guided, and **in that** the arcuate guide groove (23) and the throttle groove (22) are introduced into an endface-side upper wall section (21) of the adjusting device (11).

2. Drip chamber arrangement (2) according to claim 1, **characterized in that** the throttle groove (22) has a variable groove depth (t1, t2, t3) along the course of the groove.

3. Drip chamber arrangement (2) according to claim 1 or 2, **characterized in that** the throttle groove (22) is oriented essentially perpendicular to the longitudinal axis (L) of the drip chamber housing (5) and has an essentially circular arcuate course.

4. Drip chamber arrangement (2) according to one of the preceding claims, **characterized in that** the adjusting device (11) is fastened to a base section of the drip chamber housing (5) via a latching connection (12).

5. Drip chamber arrangement (2) according to claim 4, **characterized in that** the latching connection (12) is formed between a bead (13), which is arranged at the bottom of the drip chamber housing (5) on the end face side and extends at least in sections in the circumferential direction of the drip chamber housing (5), and a plurality of latching elements (14), which are arranged on the upper wall section (21) of the adjusting device (11) on the end face side and are latched to the bead (13) so as to be rotatable in the circumferential direction.

6. Drip chamber arrangement (2) according to one of the preceding claims, **characterized in that** a sealing element (15) is arranged between the drip chamber housing (5) and the adjusting device (11).

7. Drip chamber arrangement (2) according to claim 6, **characterized in that** the sealing element (15) is designed in such a way that it exerts a spring force on the adjusting device (11) and the drip chamber housing (5) in the axial direction.

8. Medical infusion system (1) with a drip chamber arrangement (2) and an infusion tube (3), **characterized in that** the drip chamber arrangement (2) is designed according to one of the preceding claims, the drip chamber housing (5) being connected to the infusion tube (3) in a fluid-conducting manner via the adjusting device (11).

## Revendications

1. Dispositif de chambre d'égouttage (2) pour un système de perfusion médicale (1) comportant un boîtier de chambre d'égouttage (5) avec un axe longitudinal et un passage (10) pour évacuer le liquide d'égouttage du boîtier de chambre d'égouttage (5), et comportant un dispositif de réglage (11) pour régler le débit volumétrique du liquide d'égouttage à travers le passage (10), qui peut être positionné par rapport au boîtier de la chambre d'égouttage (5) et est fixé à celui-ci dans la direction axiale et/ou radiale, dans lequel le dispositif de réglage (11) est fixé au boîtier de la chambre d'égouttage (5) et peut tourner autour de l'axe longitudinal par rapport à celui-ci, et dans lequel le dispositif de réglage est conçu de telle sorte qu'un canal de conduction de fluide (18) à l'intérieur de l'agencement de la chambre d'égouttage (2), qui est associé au passage (10) et relié à lui de manière à conduire le fluide, peut être modifié en fonction de la position de rotation relative du dispositif de réglage (11), le canal de conduction du fluide (18) comprenant une section d'une gorge d'étranglement (22) dont la section transversale varie le long de la gorge, **caractérisé en ce que** le dispositif de réglage (11) a une rainure de guidage arquée (23) dans laquelle un élément de guidage (24) fixé par rapport au boîtier de la chambre d'égouttage (5) est guidé, et **en ce que** la rainure de guidage arquée (23) et la rainure d'étranglement (22) sont introduites dans une section de la paroi supérieure (21) du dispositif de réglage (11) du côté de la face d'extrémité.

2. Dispositif de chambre d'égouttage (2) selon la revendication 1, **caractérisé en ce que** la rainure d'étranglement (22) a une profondeur de rainure variable (t1, t2, t3) le long de la trajectoire de la rainure.

3. Dispositif de chambre d'égouttage (2) selon la revendication 1 ou 2, **caractérisé en ce que** la rainure d'étranglement (22) est orientée essentiellement perpendiculairement à l'axe longitudinal (L) du boîtier de la chambre d'égouttage (5) et a un tracé essentiellement circulaire en arc de cercle.

4. Dispositif de chambre d'égouttage (2) selon l'une des revendications précédentes, **caractérisé par le fait que** le dispositif de réglage (11) est fixé à une section de base du boîtier de la chambre d'égouttage (5) par l'intermédiaire d'un raccord de verrouillage (12).

5. Dispositif de chambre d'égouttage (2) selon la revendication 4, **caractérisé en ce que** la connexion de verrouillage (12) est formée entre un bourrelet (13), qui est disposé au fond du boîtier de la chambre d'égouttage (5) du côté de la face frontale et qui s'étend au moins en sections dans la direction circonférentielle du boîtier de la chambre d'égouttage (5), et une pluralité d'éléments de verrouillage (14), qui sont disposés sur la partie supérieure de la paroi (21) du dispositif de réglage (11) du côté de la face frontale et qui sont verrouillés au bourrelet (13) de manière à pouvoir tourner dans la direction circonférentielle.

6. Dispositif de chambre d'égouttage (2) selon l'une des revendications précédentes, **caractérisé en ce qu'**un élément d'étanchéité (15) est disposé entre le boîtier de la chambre d'égouttage (5) et le dispositif de réglage (11).

7. Dispositif de chambre d'égouttage (2) selon la revendication 6, **caractérisé en ce que** l'élément d'étanchéité (15) est conçu de telle sorte qu'il exerce une force de ressort sur le dispositif de réglage (11) et le boîtier de la chambre d'égouttage (5) dans la direction axiale.

8. Système de perfusion médicale (1) avec une chambre d'égouttage (2) et un tube de perfusion (3), **caractérisé par le fait que** la chambre d'égouttage (2) est conçue selon l'une des revendications précédentes, le boîtier de la chambre d'égouttage (5) étant relié au tube de perfusion (3) par le dispositif de réglage (11) de manière à assurer la conduction du fluide.
